(19) Europäisches Patentamt
European Patent Office
Office européen des brevets

(11) Numéro de publication: **0 022 710 B1**

# (12) FASCICULE DE BREVET EUROPEEN

(45) Date de publication du fascicule du brevet: **18.04.84**

(51) Int. Cl.³: **C 07 D 243/28**, C 07 D 243/30

(21) Numéro de dépôt: **80401026.2**

(22) Date de dépôt: **08.07.80**

## (54) Procédé d'obtention de benzodiazépines-1,4.

(30) Priorité: **12.07.79 FR 7918153**

(43) Date de publication de la demande: **21.01.81 Bulletin 81/3**

(45) Mention de la délivrance du brevet: **18.04.84 Bulletin 84/16**

(84) Etats contractants désignés: **BE DE GB IT NL SE**

(56) Documents cités:
**CH - A - 573 926**
**CH - A - 579 556**
**CH - A - 581 606**
**FR - A - 2 350 346**

**Le dossier contient des informations techniques présentées postérieurement au dépôt de la demande et ne figurant pas dans le présent fascicule.**

(73) Titulaire: **SANOFI, société anonyme, 40, Avenue George V, F-75008 Paris (FR)**

(72) Inventeur: **Demarne, Henri, Le Florence-avenue Major Flandre, F-34000 Montpellier (FR)**
Inventeur: **Hallot, André, 83 rue du Juge, St Gely du Fesc (FR)**

(74) Mandataire: **de Haas, Michel et al, Cabinet Beau de Loménie 55 rue d'Amsterdam, F-75008 Paris (FR)**

EP 0 022 710 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

# Procédé d'obtention de benzodiazépines-1,4

La présente invention concerne un procédé de préparation de benzodiazépines-1,4 substituées en 3 par un radical carboxylate d'alkyle. Les benzodiazépines que l'on peut préparer selon l'invention sont les produits de formule:

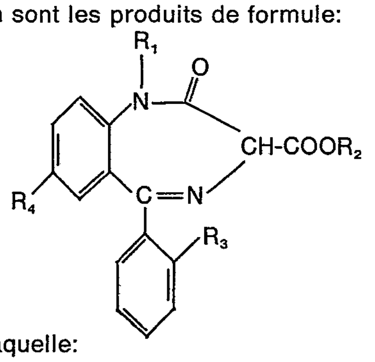

I

dans laquelle:

$R_1$ est un radical alkyle inférieur ou un atome d'hydrogène,

$R_2$ est un radical alkyle inférieur droit ou ramifié comportant de 1 à 6 atomes de carbone,

$R_3$ est un atome d'hydrogène, ou un radical halogène (chlore ou fluor par exemple) ou trifluorométhyle,

$R_4$ est un atome d'hydrogène, un radical halogène ou un radical nitro.

Il était connu, par le brevet FR-A-2 350 346, de préparer des benzodiazépines-1,4 substituées en 3 par un carboxylate d'alkyle en faisant réagir une orthoaminobenzophénone convenablement substituée avec le monochlorure monoester de l'acide aminomalonique dans lequel la fonction amine a été bloquée par un groupe benzyloxycarbonyle. Un tel procédé a conduit lors de son utilisation à un certain nombre de difficultés expérimentales et à une insuffisance de rendement. Il a été trouvé dans la présente invention que le remplacement, dans le procédé du brevet FR-A-2 350 346 du groupement bloqueur benzyloxycarbonyle par un groupement méthyloxycarbonyle, permettait de surmonter ces difficultés expérimentales.

Le procédé de préparation des benzodiazépines de formule (I) selon la présente invention est caractérisé en ce que, dans une première étape, on réalise la réaction d'une benzophénone substituée de formule:

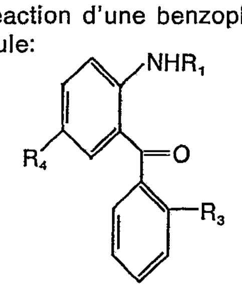

sur un dérivé d'aminomalonate d'alkyle, dans lequel le groupement amine a été bloqué, ladite première étape étant réalisée soit avec le monochlorure monoester de l'acide aminomalonique de formule:

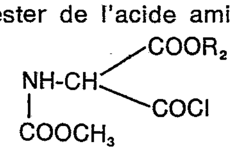

la réaction avec la benzophénone substituée étant effectuée entre −5 et 0 °C dans un milieu pyridine-eau et le produit obtenu étant traité par un hydracide en milieu solvant, soit avec l'oxazolidènedione de formule:

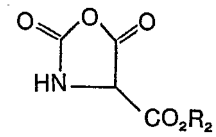

la réaction étant effectuée en présence d'acide chlorhydrique dans un solvant anhydre, de préférence un solvant halogéné, le produit obtenu étant ensuite dans l'un et l'autre cas traité de façon connue par un mélange acide acétique/acétate de sodium, en vue de réaliser la cyclisation dudit produit.

Le dérivé de l'aminomalonate d'alkyle, dans lequel le groupement aminé a été convenablement bloqué, utilisable selon l'invention est choisi parmi:

– le monochlorure monoester d'un aminomalonate dans lequel le groupement aminé a été bloqué sous forme de carbamate; un tel réactif a pour formule:

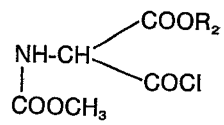

dans laquelle $R_2$ a la signification mentionnée cidessus

– l'oxazolidinedione de formule

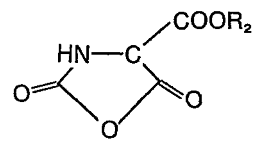

qui est en fait l'anhydride interne de l'acide

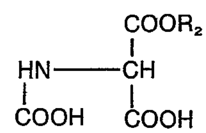

dans lequel le radical carboxylique porté par l'atome d'azote joue le rôle d'élément bloqueur du groupement aminé de l'acide malonique. Lorsqu'on utilise le monochlorure monoester d'un aminomalonate à groupement aminé bloqué sous forme de carbamate, les réactions peuvent être représentées comme suit:

on fait réagir ledit monochlorure de formule

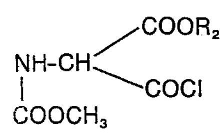

(4)

sur l'aminobenzophénone (5) dans un mélange pyridine-eau entre −5 et 0 °C pour isoler le composé (6) répondant à la formule suivante:

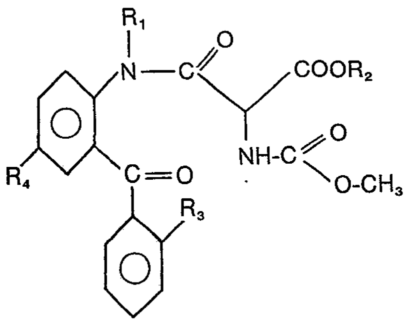

(6)

Dans le composé (6), le groupe méthoxycarbonyle est éliminé par un hydracide dans un solvant approprié, par exemple l'acétate d'éthyle à ébullition. On isole le composé 7 répondant à la formule suivante:

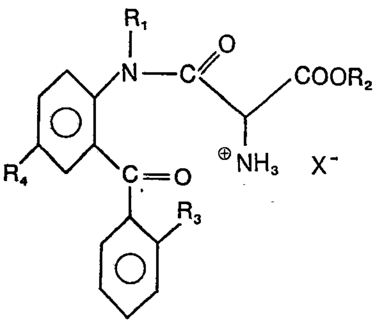

(7)

Le composé (7) est cyclisé en composé I par un mélange d'acide acétique/acétate de sodium à l'ébullition.

Lorsqu'on utilise l'oxazolidinedione de formule

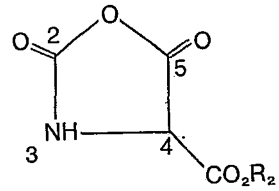

II

on fait réagir ledit produit sur une benzophénone (5) selon la réaction:

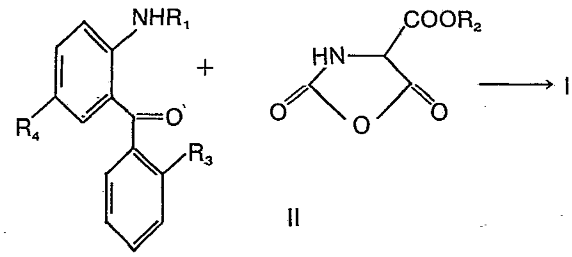

II

5

Dans cette réaction, on fait réagir le composé II sur la benzophénone (5), dans un solvant anhydre (halogéné par exemple), en présence d'acide chlorhydrique gazeux, à température ambiante durant quelques heures.

On chasse les solvants et reprend le résidu solide par mélange (acide acétique, acétate de sodium) à l'ébullition.

L'oxazolidinedione de formule II peut être préparé à partir de l'aminomalonate d'alkyle correspondant 1 selon la suite de réactions ci-dessous: (décrite par ailleurs: JACS 75, 78 (1953) Exp: 8, 98 (1952)

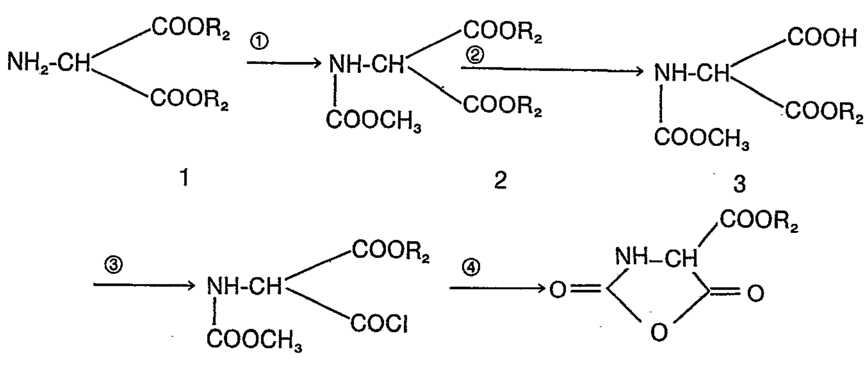

1 2 3

4 II

Réaction 1 : Action sur 1 du chloroformiate d'alkyle correspondant

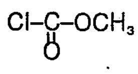

Réaction 2 : Hémisaponification de l'ester 2 par la soude

Réaction 3 : Action sur l'hémiester 3 du pentachlorure de phosphore

Réaction 4 : Formation du composé II par perte du chlorure d'alkyle $CH_3Cl$.

Les exemples non limitatifs ci-après illustrent l'invention.

EXEMPLE 1

Préparation du chloro-7 (chloro-2 phényl)-5 éthoxycarbonyl-3 oxo-2 dihydro-2,3 1H-benzodiazépine-1,4 par utilisation d'oxazolidinedione.

($R_1$ = H; $R_2$ = $C_2H_5$; $R_3$ = Cl; $R_4$ = Cl).

Dans une solution refroidie au bain de glace contenant 3 g de dichloro-2,5 amino-2 benzophénone dans 20 $cm^3$ de chlorure de méthylène, faire barboter pendant 20 min un courant de gaz chlorhydrique sec, ajouter une solution de 5,88 g d'oxazolidinedione-2,5 carboxylate d'éthyle-3 dans 50 $cm^3$ de chlorure de méthylène et laisser 48 h à température ambiante. Verser sur glace/ammoniaque, décanter la phase organique, sécher sur sulfate de sodium, évaporer à sec sous vide. Le résidu est repris dans 25 $cm^3$ d'acide acétique glacial et porté à reflux 1 h. Verser sur glace/ammoniaque, extraire au chlorure de méthylène, laver à l'eau, sécher, évaporer sous vide le solvant. Reprendre le résidu dans l'éther, laisser cristalliser.

Poids : 2,2 g, Fk : 138–140 °C.

EXEMPLE 2

Préparation du chloro-7 (fluoro-2 phényl)-5 éthoxycarbonyl-3 oxo-2 dihydro-2,3 1H-benzodiazépine-1,4 par utilisation d'un monochlorure monoester d'un aminomalonate à groupement aminé bloqué sous forme de carbamate.

a) Ethoxycarbonyl-3 méthoxycarbonylamino-3 aza-1 oxo-2 propyl)-1 (fluoro-2 benzoyl)-2 chloro-4 benzène.

(6) ($R_1$ = H; $R_2$ = $C_2H_5$; $R_3$ = F; $R_4$ = Cl).

A une solution agitée de 20 g d'acide (carbométhoxyamino)-2 malonate d'éthyle (0,086 mol) dans 100 $cm^3$ de chlorure de méthylène, refroidie à $-10$ °C, ajouter 21,5 g de pentachlorure de phosphore (1,05 mol) par petites portions en 15 min. Laisser 15 min à $-10$ °C. Ajouter alors goutte à goutte une solution de 21,4 g d'amino-2 chloro-5 fluoro-2' benzophénone dans 100 $cm^3$ de chlorure de méthylène, laisser 30 min à 0 °C et ajouter, en maintenant la température à 0 °C sous agitation, 200 $cm^3$ d'une solution à 40% dans l'eau de carbonate de potassium. Laisser 1 h au contact. Décanter la phase organique, laver à l'eau, sécher et évaporer à sec sous vide. Reprendre le résidu dans l'éther isopropylique. Laisser cristalliser.

Poids : 27 g de 6, Fk : 106–108 °C.

b) Bromhydrate d'(amino-3 éthoxycarbonyl-3 aza-1 oxo-2 propyl)-1 (fluoro-2 benzoyl)-2 chloro-4 benzène.

(7) ($R_1$ = H; $R_2$ = $C_2H_5$; $R_3$ = F; $R_4$ = Cl).

Une solution de 10 g de 6 dans l'acétate d'éthyle est portée à reflux. Faire barboter un courant d'acide bromhydrique pendant 1 h en maintenant le reflux. Laisser refroidir. Filtrer, laver à l'éther.

Poids : 12 g, Fk : 208–210 °C.

c) Chloro-7 (fluoro-2 phényl)-5 éthoxycarbonyl-3 oxo-2 dihydro-2,3 1H-benzodiazépine-1,4.

($R_1$ = H; $R_2$ = $C_2H_5$; $R_3$ = F; $R_4$ = Cl).

Porter à reflux 2 h un mélange de 5 g de 7, 2,5 g d'acétate de sodium, 10 $cm^3$ d'eau, 75 $cm^3$ d'acide acétique. Chasser le maximum d'acide acétique sous vide, reprendre dans l'eau, alcaliniser avec de l'ammoniaque, extraire au chloroforme, sécher, évaporer, reprendre le résidu dans l'éther, laisser cristalliser.

Poids : 2,9 g, Fk : 200 °C.

A titre d'exemple, les composés I suivants ont pu être obtenus par l'une ou l'autre de ces deux voies.

| | $R_1$ | $R_2$ | $R_3$ | $R_4$ | Fk °C |
|---|---|---|---|---|---|
| Utilisant l'oxazolidine (II) | H | $C_2H_5$ | H | Cl | 242 |
| | H | $C_2H_5$ | F | Cl | 200 |
| | H | $C_2H_5$ | Cl | Cl | 140 |
| | $CH_3$ | $C_2H_5$ | H | Cl | 190 |
| | H | $C_2H_5$ | $CF_3$ | H | 183 |
| Utilisant directement (*4*) | H | $C_2H_5$ | F | Cl | 110 |

## Revendication

Procédé pour la préparation de benzodiazépines-1,4 substituées en 3 par un radical carboxylate d'alkyle de formule:

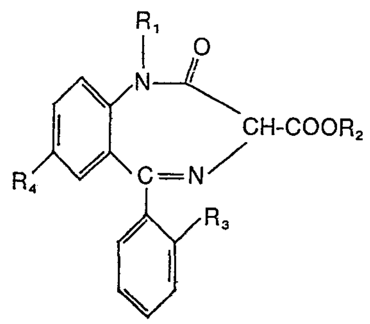

I

dans laquelle:

$R_1$ est un radical alkyle inférieur ou un atome d'hydrogène,

$R_2$ est un radical alkyle inférieur droit ou ramifié comportant de 1 à 6 atomes de carbone,

$R_3$ est un atome d'hydrogène, ou un radical halogène (chlore ou fluor par exemple) ou trifluorométhyle,

$R_4$ est un atome d'hydrogène, un radical halogène ou un radical nitro, procédé caractérisé en ce que, dans une première étape, on réalise la réaction d'une benzophénone substituée de formule:

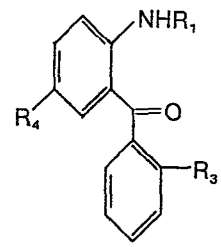

dans laquelle $R_1$, $R_3$ et $R_4$ ont les significations mentionnées ci-dessus, sur un dérivé d'aminomalonate d'alkyle, dans lequel le groupement amine a été bloqué, ladite première étape étant réalisée soit avec le monochlorure monoester de l'acide aminomalonique de formule:

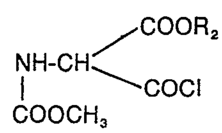

dans laquelle $R_2$ a la signification mentionnée ci-dessus, la réaction avec la benzophénone substituée étant effectuée entre −5 et 0 °C dans un milieu pyridine eau et le produit obtenu étant traité par un hydracide en milieu solvant, soit avec l'oxazolidènedione de formule:

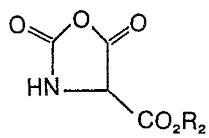

dans laquelle $R_2$ a la signification mentionnée ci-dessus, la réaction étant effectuée en présence d'acide chlorhydrique dans un solvant anhydre, de préférence un solvant halogéné, le produit obtenu étant ensuite dans l'un et l'autre cas traité de façon connue par un mélange acide acétique/acétate de sodium, en vue de réaliser la cyclisation dudit produit.

**Claim**

Process for the preparation of 1,4-benzodiazepines substituted in 3 by an alkyl carboxylate radical of formula:

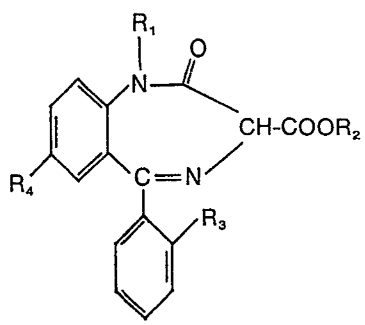

I

wherein:

$R_1$ is a lower alkyl radical or a hydrogen atom,

$R_2$ is a straight or branched lower alkyl radical comprising from 1 to 6 carbon atoms,

$R_3$ is a hydrogen atom, or a halogen radical (for example chlorine or fluorine) or trifluoromethyl,

$R_4$ is a hydrogen atom, a halogen radical or a nitro radical, process characterized in that, in a first step, is carried out a reaction of a substituted benzophenone of formula:

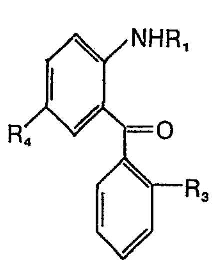

wherein $R_1$, $R_3$ and $R_4$ are as indicated above with a derivative of alkyl aminomalonate, wherein the amine group was blocked, said first step being carried out either with monoester monochloride of the aminomalonic acid of formula:

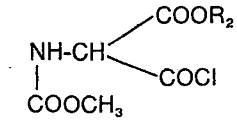

wherein $R_2$ is as indicated above, the reaction with the substituted benzophenone being carried out between −5 and 0 °C in a pyridin water medium and the product obtained therefrom being treated by a hydracide in a solvent medium, either with oxazolidenedione of formula:

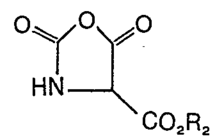

wherein $R_2$ is as indicated above, reaction being carried out in the presence of hydrochloric acid in an anhydrous solvent, preferably a halogenous solvent, the product obtained being thereafter treated in both cases in known manner with a mixture of acetic acid/sodium acetate, with the view to obtain the cyclization of said product.

**Patentanspruch**

Verfahren zur Herstellung von an der Position 3 durch eine Alkylcarboxylatgruppe substituierten 1,4-Benzodiazepinen der Formel

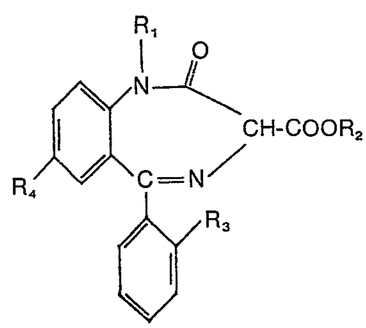

I

worin:

$R_1$ eine Niedrigalkylgruppe oder ein Wasserstoffatom ist,

$R_2$ eine gerade oder verzweigte Niedrigalkylgruppe mit 1 bis 6 Kohlenstoffatomen ist,

$R_3$ ein Wasserstoffatom oder ein Halogenradikal (beispielsweise Chlor oder Fluor) oder Trifluormethyl ist,

$R_4$ ein Wasserstoffatom, ein Halogenradikal oder eine Nitrogruppe ist,

welches Verfahren dadurch gekennzeichnet ist, dass in einer ersten Stufe die Reaktion eines substituierten Benzophenons der Formel

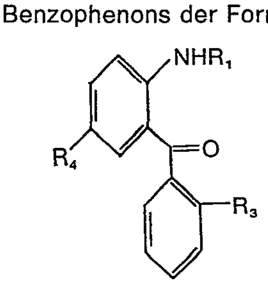

worin $R_1$, $R_3$ und $R_4$ obige Bedeutung haben, über ein Aminomalonsäurealkylesterderivat, in dem die Aminogruppe geschützt vorliegt, stattfindet, welche erste Stufe entweder mit einem Aminomalonsäuremonochloridmonoester der Formel

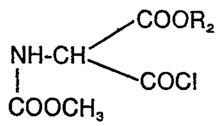

worin $R_2$ obige Bedeutung hat, realisiert wird, wobei die Umsetzung mit dem substituierten Benzophenon zwischen −5 und 0 °C in Pyridin-Wasser-Milieu ausgeführt und das erhaltene Produkt in einem Lösungsmittel mit einer Wasserstoffsäure behandelt wird, oder mit einem Oxazolidindion der Formel

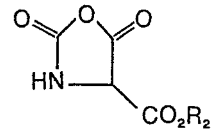

worin $R_2$ obige Bedeutung hat, realisiert wird, welche Umsetzung in Gegenwart von Salzsäure in einem wasserfreien Lösungsmittel, vorzugsweise einem halogenierten Lösungsmittel, ausgeführt wird, wobei das erhaltene Produkt danach in beiden Fällen auf bekannte Weise mit einer Mischung aus Essigsäure/Natriumacetat behandelt wird, um die Zyklisierung des Produkts zu bewirken.